Europäisches Patentamt

European Patent Office

Office européen des brevets

(19)

(11) Publication number: **0 455 597 A1**

# EUROPEAN PATENT APPLICATION

(12)

(21) Application number: 91810315.1

(22) Date of filing: 26.04.91

(51) Int. Cl.⁵: **C12N 5/00, C12N 5/04**

(30) Priority: 30.04.90 GB 9009674

(43) Date of publication of application:
06.11.91 Bulletin 91/45

(84) Designated Contracting States:
AT BE CH DE DK ES FR GB GR IT LI LU NL SE

(71) Applicant: SANDOZ LTD.
Lichtstrasse 35
CH-4002 Basel (CH)
BE CH DK ES FR GB GR IT LI LU NL SE
Applicant: SANDOZ-PATENT-GMBH
Humboldtstrasse 3
W-7850 Lörrach (DE)
DE
Applicant: SANDOZ ERFINDUNGEN
VERWALTUNGSGESELLSCHAFT M.B.H.
Brunner Strasse 59
A-1235 Vienna (AT)
AT

(72) Inventor: Kreuger, Marc
Goudsjidje 5
NL-1602 KL Enkhuizen (NL)
Inventor: Van Holst, Gerrit-Jan
De Gouw 8
NL-1602 DN Enkhuizen (NL)
Inventor: Kool, Arnoldus Johannes
Kerkeland 74
NL-1602 LJ Enkhuizen (NL)

(54) A method for stimulating growth, division or embryogenesis of plants in in vitro culture.

(57) A method of stimulating the growth division or somatic embryogenesis of plant cells in in vitro culture by applying to the culture medium an effective amount of AGPs and culture media comprising such AGPs.

EP 0 455 597 A1

Jouve, 18, rue Saint-Denis, 75001 PARIS

The present invention relates to arabinogalactan-proteins (AGPs) and their use for in vitro cultivation of plants.

It is known that some plant cells have the potential to differentiate into whole plants when cultured in appropriate basic synthetic media. Such media typically comprise inorganic salts, a carbon source such as sucrose, thiamine and inositol. Examples of basic media commonly used are those described by Murashige and Skoog, Lindsmaier and Skoog, by Gamborg (B5 medium), and Chu et al (N6 medium).

The composition of said basic media may be modified to optimise the growth of the particular plant cells employed. Almost all plant cells require plant hormones, e.g. auxin or auxin-like compounds such as indole acetic acid, indole butyric acid, naphthalene acid or 2,4-D, and/or a cytokinin such as benzyl adenine, zeatin, kinetin and the like. To secure optimal growth it may be advantageous to add vitamins such as nicotinic acid and pyridoxine.

The primary type of growth that is usually obtained when explanting plant tissue to a synthetic medium is the callus. The capacity of plant tissue to form a callus depends i.a. on the type of tissue employed and on the presence of plant growth hormones. Plant tissue in general suitable for the preparation of callus include explants from stems, leaves, petals, hypocotyl sections, apical meristems, embryos and the like.

The artificial growth media may be solidified, e.g. with the aid of agar. Once established, the callus exhibits a rapid growth rate and must be subcultured. This is conveniently done in a suspension culture comprising a basic synthetic medium, a carbon source and plant growth hormones. The cells of such subcultures may be employed to generate somatic embryos, e.g. by subculturing on basic synthetic medium to which a carbon source, but no or low concentrations of growth hormons have been added. This medium may or may not be solidified. In incidental cases it has been suggested that the addition of complex non-defined mixtures of compounds such as coconut milk, rice-water or potato extracts stimulate cell growth, plant cell division, plant cell differentiation and/or embryo induction.

Whilst vegetative propagation of plants has great potential for the development and mass production of plants and is routinely used, in particular for the production of orchids from somatic orchid cells, it has been found only limited application for a vast majority of plant species.

Vegetative propagation via shoot formation (organogenesis) involves the steps of placing explants on a solid nutrient medium, regenerating shoots and obtaining plants from the shoots. This technique is very labour intensive and for economical reasons only meaningful for a limited number of ornamentals.

Also the alternative meristem culture technique has primarily commercial application in the production of ornamentals.

Vegetative propagation via plant somatic embryos (embryogenesis) is substantially less labour intensive than that required for vegetative propagation via shoot formation but suffers from the drawback that the efficiency of somatic embryogenesis is for most plant species unsatisfactory (exceptions are carrot, celery, alfalfa and cucumber cell cultures) and that for most plant species it is not known how to induce or enhance embryogenesis of plant cells.

The ability to stimulate embryogenesis in plant cells is therefore one of the most critical factors in the commercial use of plant tissue culture techniques for the purpose of vegetative plant propagation.

Whether a culture is embryogenic or non-embryogenic is established by microscopic examination for the presence of proembryogenic masses or embryoids. From the embryogenic culture somatic embryos can be generated.

It has now been found that AGPs are suitable for inducing or enhancing the growth and the somatic embryogenesis of plant cells.

The invention therefore provides a method of stimulating the growth and division or embryogenesis of plant cells or the growth and division of plant cells derived from protoplasts in plant tissue culture medium which comprises applying to plant cells or protoplast in a plant tissue, cell or protoplast culture medium an effective amount of AGPs. The method is generally applicable, including for anther and microspore cultures. The method of the invention is therefore also indicated for use in the production of dihaploid plants or so-called fast inbreds.

The terms plant tissue, cell and protoplast culture medium refers to any such conventional culture medium suitable for culture of plant tissue, plant cells and plant protoplasts.

AGPs are proteoglycans having covalently attached to their protein moiety oligo-saccharide and/or polysaccharide side chains. Their small protein portion contains hydroxyproline. AGPs are found in higher plants and in many of their secretions (Fincher et al, 1983) . It has been shown that different organs of Lycopersicon peruvianum have different AGPs (Van Holst and Clarke, 1986) suggesting a role of AGPs in cell differentiation and plant development.

The type and amount of AGP to be employed will conveniently be selected in accordance with the purpose of the use. Where it is intended to just induce or enhance cell division or growth, the AGPs to be employed may be of any source, including commercially available AGPs in the form of gum Arabic. It is however advantageous to employ AGPs derived from the same species as the species of which to induce or enhance cell division or growth. Where it is desired to induce or enhance somatic embryogenesis of plant cells, it is advantageous to employ embryogenic AGPs, i.e. AGPs obtained from embryogenic plant cultures, from zygotic embryos, zygotic embryosacs or from seeds, in particular from seeds. It is advantageous to employ embryogenic AGPs derived from the same species as the species of which to induce or enhance somatic embryogenesis.

The amount of AGPs to be applied will depend on various factors including the type of plant tissue employed, and the purpose of their use.

Where a plant suspension culture is employed, satisfactory results will, in general, be obtained with concentrations in the range from 0.01 to 100 mg AGP per litre culture medium.

For the purpose of embryogenesis induction or enhancement a suitable concentration lies, in general, in the range of from 0.1 to 20 mg preferably of from 1 to 10 mg embryogenic AGPs per litre culture medium. For growth and division enhancement or growth induction of plant cells, the preferred concentrations are, in general, in the range of from 20 to 100 mg AGP, in particular 25 to 50 mg AGP per litre culture medium.

Though the chemical composition of embryogenic AGPs may vary from plant species to plant species, the embryogenesis inducing or enhancing effect is not species specific. It is therefore possible to induce or enhance embryogenesis of plant cells from one plant species with the aid of embryogenic AGPs obtained from a different plant species. However, it is advantageous to employ embryogenic AGPs derived from the same species. It has been observed that a particular fraction of the embryogenic AGPs is responsible for the embryogenesis inducing or enhancing effect. It is in general not necessary to isolate that particular fraction, but it is advantageous to do so. Methods for fractionation of AGPs are known, they include antibody precipitation, affinity chromatography, ion exchange chromatography, electrophoresis and the like. The growth or embryogenesis inducing or enhancing effect of AGPs or of a particular fraction thereof can be determined by tests. According to a preferred embodiment of the invention the embryogenesis of the plant cells is induced with a fraction of embryogenic AGPs giving an optimum effect.

The invention also provides plant tissue, plant cell and plant protoplast culture media comprising from 0.01 to 100 mg AGPs per litre culture medium. The following Examples illustrate the invention.

**Example 1: Preparation of somatic embryos from Daucus carota L. Seeds**

Seeds of Daucus carota L. are surface sterilised and germinated on Gamborg's B-5 medium (see Table 1) supplemented with 0.6 % agar, at 22.5° C in the dark. Cell cultures are derived from ten days old sliced hypocotyls. About two grams of hypocotyl parts are incubated in 50 ml Gamborg's B-5 medium supplemented with 2 μM 2,4-dichlorophenoxyacetic acid (2,4-D), in a 16 hr day light period at 22.5°C, on a rotary shaker at 100 rpm. The cultures are subcultured every two weeks. After four weeks of subculture the cultures are embryogenic or non-embryogenic, depending on the cultivar employed. Non-embryogenic cultures can also be made by rapid subculturing for a prolonged time (minimal two months). The formation of somatic embryos is induced by selecting cells and aggregates that pass a 150 μm sieve and are retained by a 50 μm sieve, and subculturing said cell fraction in auxin free B-5 medium at a cell density of 2000 cells/ml (De Vries et al, 1988).

**Table 1: Gamborg's B-5 medium:**

| Components | mg/l |
|---|---|
| CoCl2.6H20 | 0.025 |
| CuSO4.5H20 | 0.025 |
| FeNaEDTA | 40.00 |
| H3B03 | 3.00 |
| KI | 0.75 |
| MnSO4.H20 | 10.00 |
| Na2MoO4.2H20 | 0.25 |
| ZnSO4.7H20 | 2.00 |
| KNO3 | 2500.00 |
| CaCl2 | 113.23 |
| NaH2PO4 | 150.00 |
| (NH4)2SO4 | 134.00 |
| MgSO4.7H20 | 250.00 |
| inositol | 100.00 |
| nicotine acid | 1.00 |
| pyridoxine HCl | 1.00 |
| thiamine HCl | 1.00 |
| sucrose | 20000.0 |

**Example 2: Isolation of AGPs**

2.1 From embryogenic cultures.

AGP fractions can be isolated from the culture medium of Example 1 or extracted from the cells in an embryogenic culture.

Plant cells are collected by centrifugation (200 g, 3 minutes) from the culture according to test example 1, one week after subculturing. The cells are incubated in 4 % sucrose in water for one hour on the rotary shaker to remove AGPs from the cells. Cells are then removed by centrifugation at 200 g for 3 minutes. AGPs are then isolated by adding to the cell-free medium or to the eluted AGPs one tenth volume of 1.5 M NaCl and 0.75 g/l of 1,3,5-tris[4-β-D-glucopyranosyl-oxyphenyl-azo]2,4-trihydroxybenzene (hereinafter designated the β-glucosyl Yariv reagent; Yariv et al. 1967). The AGPs are precipitated by cooling at 4°C for three hours and the precipitate is centrifugated at 10000 g for 10 minutes. The pellet is then dissolved in water and NaCl is added to 0.15 M to reprecipitate the complex. The precipitate is centrifugated at 10000 g for 10 minutes and the complex is dissolved in one tenth of the original volume of 0.1 M NaOH comprising 10 % NaCl. The AGPs are separated from the β-glucosyl Yariv reagent on a Sephadex G-50 column, equilibrated with 0.1 M NaOH comprising 10 % NaCl, and desalted on another Sephadex G-50 column, equilibrated with water to yield 22 mg AGP/L medium. The AGP preparations are free of other proteins. The purified AGPs can be further fractionated by electrophoresis or ion-exchange chromatography.

## 2.2 From seeds, zygotic embryos or embryosacs

Embryogenic AGPs are isolated from Daucus carota seeds, zygotic embryos or zygotic embryosacs by grinding 4 grams of tissue in liquid nitrogen in a mortar and extracting them with 23 ml of extraction buffer (50 mM tris-HCl [pH 8.0], 10 mM EDTA, 2 mM $Na_2S_2O_5$, 1 % v/v Triton X-100). Cell debris is removed by centrifugation (10000 g, 5 minutes at 4°C). AGPs are precipitated by adding five volumes of ethanol. After incubation overnight at -20°C, the AGPs are pelleted by centrifugation at 10000 g at 4°C for 10 minutes. The pellet is resuspended in 50 mM tris-HCl (pH 8.0). From this crude extract, AGPs are isolated according to test example 2.1 described above by precipitation with β-glucosyl Yarif reagent. The AGP concentration is determined by the single radial diffusion method of Van Holst and Clarke (1985); 0.12 mg AGP per gram seeds was isolated.

## 2.3 From suspension cultures of Lycopersicon esculentum L.

Analogous to the procedure of Example 2.1, AGPs are isolated from the culture medium of tomato suspension.

## 2.4 From seeds of Cucumis sativus L.

Analogous to the procedure of Example 2.2, embryogenic AGPs are obtained from seeds of Cucumis sativus L. _

## 2.5 From seeds of Brassica olevacea

Analogous to the procedure of Example 2.2, embryogenic AGPs are obtained from seeds of Brassica oleracea.

## Example 3: Induction or stimulation of embryo formation in in vitro cultures by addition of embryogenic AGPs

a) Addition of embryogenic AGPs (5.0 mg/l) isolated from seeds of Daucus carota to a non-embryogenic culture of Daucus carota induced to form embryos, makes this culture embryogenic. Morphologically perfect embryos are formed, whereas the control experiments (no AGPs added) show no embryo formation.

b) Addition of AGPs (0.5 and 5.0 mg/l) isolated from seeds of Daucus carota to an embryogenic culture of Daucus carota induced to form embryos, results in a faster development of the somatic embryos. After 18 days of culture almost twice as many torpedo shaped embryos are formed in the complemented cultures as compared to the control experiments (no AGPs added).

c) The embryogenic Daucus carota culture is washed with 4 % sucrose in water to remove AGPs from the cells. The cells are then subcultured in hormone-free medium with or without added AGPs to induce embryoformation. When AGPs are added (1-5 mg/l, e.g. 1 mg/l) isolated from seeds of Daucus carota more somatic embryos are formed than in experiments with no AGPs added.

d) Crossed electrophoresis of AGPs indicates that embryogenic and non-embryogenic cultures have different arabinogalactanproteins. A part of the AGPs present in embryogenic Daucus carota cultures, are absent in non-embryogenic Daucus carota cultures.

Addition of the β-glucosyl-Yariv reagent in a concentration of 1 mg/l, to a Daucus carota L. suspension culture induced to start embryogenesis, results in a total block of embryogenesis. Addition of α-galactosyl Yariv reagent, 1,3,5-tris[4-α-D-galactopyranosyl-oxyphenyl azo]2,4-6-trihydroxy-benzene, known not to bind to AGPs in a concentration of 1 mg/l does not block somatic embryo developments. This shows that AGPs are essential for somatic embryogenesis to take place.

e) Embryogenic AGPs are added to two different cultures of young Daucus carota cells in the hormone containing medium according to Example 1 (conc. 10 mg AGPs per litre medium). These cultures were potentially embryogenic, but were at that time unable to produce large amounts of embryos. The added AGPs were isolated from Daucus carota and Cucumis sativus seeds and from cell cultures of Daucus carota and Lycopersicon esculentum. During the subculture the percentage of embryogenic cells was determined by counting single cells and small aggregates (aggregates of less than 15 cells) and discriminating between the embryogenic cells (dense, highly cytoplasmic) and the non-embryogenic cells (large, highly vacuolated) (De Vries et al., 1988). The number of embryogenic cells is counted 8 days and 15 days after addition of AGPs, and the result compared with that of an untreated standard. The results indicate that embryogenic AGPs are suitable for enhancing the embryogenesis of plant cells. When AGPs were added the percentage of embryogenic cells increased more than in control experiments where no AGPs were added. Even AGPs isolated from different species were able to increase the percentage of embryogenic cells, but AGPs isolated from the same species were able to increase the percentage of embryogenic cells the most.

f) Daucus carota cells were cultured on the hormone free medium in which the embryos develop. The same AGPs, at concentrations of 1 and 10 mg per litre medium, were added as mentioned in Example 3 (e). More somatic embryos were formed when the AGPs were added than in control experiments where no AGPs were added.

g) Daucus carota seed AGPs were fractionated by ion exchange chromatography in three fractions. In an experiment as described in Example 3(e) two of these fractions were able to increase the percentage of embryonic cell to a higher degree than the unfractionated Daucus carota seed AGPs.

## Example 4: Stimulation of growth in in vitro cultures by addition of AGPs

a) When 2,4-D containing cell cultures of Daucus carota L are diluted growth is inhibited if the cell density is 10000 cells/ml or less. The cell growth of such diluted cell cultures is stimulated by the addition of AGPs (25 and 50 mg/l) isolated from Daucus carota and Lycopersicon esculentum cell cultures and by the addition of Arabic Gum (an AGP isolated from the tree Acacia senegal L.). These results show that AGPs can stimulate growth even when the AGPs are isolated from a different species. However, AGPs isolated from the same species were the most effective in stimulating growth. The stimulation of the growth of the cell cultures can be dramatically. When no AGPs are added in the diluted carrot cell cultures there is hardly any growth. When AGPs are added, the growth rate increases and can be compared with undiluted cell cultures. A similar effect is observed in cell cultures of other species (Cucumis sativus L. and Lycopersicon esculentum L.).

b) Protoplasts of Brassica oleracea L. were cultured in the presence of 10 to 30 mg/l Brassica oleracea seed AGPs, the percentage of dividing cells derived from the protoplasts increased from 2 % (no AGPs added) to 10 %. This increase also resulted in the production of more microcalli. Analogously, the addition of AGPs from carrot suspensions and carrot seeds resp. also gave an increase in the percentage of dividing cells, from 2 % (no AGPs added) to 4 % when added to Brassica protoplasts.

## References

Fincher, G.B, B.A. Stone and A.E. Clarke (1983) Ann. Rev. Plant Physiol. 34:47-70. Arabinogalactan-proteins; Structure, biosynthesis and function.

Van Holst, G.J. and A.E. Clarke (1985) Analytical Biochemistry 148;446-450. Quantification of arabinogalactan-protein in plant extracts by single radical gel diffusion.

Van Holst, G.J. and A.E. Clarke (1986) Plant Physiol. 80: 786-789, Organ-specific arabinogalactan-protein of Lycopersicon peruvianum (Mill) demonstrated by crossed-electrophoresis.

De Vries , S.C., H. Booij, R. Janssens, R. Vogels, L. Saris, F. LoSchiavo, M. Terzi and A. van Kammen (1988) Genes and Development 2:462-476, Carrot somatic embryogenesis depends on the phytohormone-controlled presence of correctly glycosylated extracellular proteins.

Yariv, J, H. Lis and E. Katchalski (1967) Biochem. J.105; Ic., Precipitation of arabic acid and some seed polysaccharides by glycosylphpenylazo dyes.

## Claims

1. A method of inducing or stimulating embryogenesis of plant cells in in vitro plant cell or tissue culture which comprises applying to the plant cell or tissue culture medium and effective amount of AGPs.

2. A method of stimulating the growth and division of plant cells or cells obtained from protoplasts or plant tissue in in vitro plant tissue, cell or protoplasts culture which comprises applying to plant cells or plant tissue or protoplasts in a plant tissue, cell or protoplasts culture medium an effective amount of AGPs.

3. The method of Claim 1 or 2, comprising applying from 0.01 to 100 mg AGP per litre culture medium.

4. The method of Claim 1 or 3, comprising employing AGPs of the same plant species as the cells or tissue in the in vitro plant cell or tissue culture.

5. The method of Claim 1, 3, or 4 comprising applying embryogenic AGPs.

6. The method of Claim 5, wherein the embryogenic AGPs are from embryogenic plant cultures, embryos, zygotic embryosacs or seeds.

7. The method of Claim 6, wherein the embryogenic AGPs are a specific fraction of the total of AGPs from embryogenic plant culture, embryos, zygotic embryosacs or seeds.

8. The method of Claims 5 to 7, employing 0.1 to 20 mg embryogenic AGPs per litre culture medium.

9. The method of Claim 2 comprising of employing AGPs of the same plant species as the cells tissue or protoplasts in the in vitro plant cell, tissue or protoplast culture.

10. The method of Claim 2 or 9, wherein the AGPs are a specific fraction of the total of AGPs extractable from plant tissue or plant cell or culture medium.

11. The method of Claim 2, 9 or 10 employing 20 to 100 mg AGPs per litre culture medium.

12. The method of Claims 1 to 11, wherein the AGPs are from Daucus.

13. The method of Claims 1 to 11, wherein the AGPs are from Lycopersicon.

14. The method of Claims 1 to 11, wherein the AGPs are from Cucumis.

15. The method of Claims 1 to 11, wherein the AGPs are from Brassica.

16. The method of Claims 1 to 11, wherein the AGPs are from Acacia.

**European Patent Office**

## EUROPEAN SEARCH REPORT

Application Number

EP    91 81 0315

| | DOCUMENTS CONSIDERED TO BE RELEVANT | | |
|---|---|---|---|
| Category | Citation of document with indication, where appropriate, of relevant passages | Relevant to claim | CLASSIFICATION OF THE APPLICATION (Int. Cl.5) |
| D,Y | GENES AND DEVELOPMENT vol. 2, 1988, COLD SPRING HARBOR US pages 462 - 476; S.C.de Vries et al.: "Carrot somatic embryogenesis depends on the phytohormone-controlled presence of correctly glycosylated extracellular proteins" * the whole document * | 1, 2 | C12N5/00 C12N5/04 |
| Y | EP-A-49632 (CHLORELLA INDUSTRY CO.,LTD.) * page 4, lines 11 - 33; claims 1-8 * | 1, 2 | |
| D,A | ANNUAL REVIEW OF PLANT PHYSIOLOGY vol. 34, 1983, PALO ALTO US pages 47 - 70; G.B.Fincher et al.: "Arabinogalactan-proteins:Structure,Biosynthesis and Function" * page 65, lines 8 - 36 * | 1, 2 | |
| | | | **TECHNICAL FIELDS SEARCHED (Int. Cl.5)** C12N A01H |

The present search report has been drawn up for all claims

| Place of search | Date of completion of the search | Examiner |
|---|---|---|
| BERLIN | 27 AUGUST 1991 | GURDJIAN D. |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document